(19) Europäisches Patentamt
European Patent Office
Office européen des brevets

(11) EP 0 804 277 B1

(12) **FASCICULE DE BREVET EUROPEEN**

(45) Date de publication et mention de la délivrance du brevet: **18.11.1998 Bulletin 1998/47**

(21) Numéro de dépôt: **96901043.8**

(22) Date de dépôt: **11.01.1996**

(51) Int. Cl.$^6$: **B01D 61/44**, B01D 61/50, C07C 303/02

(86) Numéro de dépôt international: **PCT/FR96/00042**

(87) Numéro de publication internationale: **WO 96/22154 (25.07.1996 Gazette 1996/34)**

(54) **REGENERATION D'ACIDES, NOTAMMENT D'ACIDES ORGANIQUES FORTS, PAR ELECTRODIALYSE AU MOYEN DE MEMBRANES BIPOLAIRES**

ELEKTRODIALYTISCHE REGENERIERUNG VON SÄUREN, INSBESONDERE STARKER ORGANISCHER SÄUREN, UNTER VERWENDUNG BIPOLARER MEMBRANEN

REGENERATION OF ACIDS, IN PARTICULAR OF STRONG ORGANIC ACIDS, BY ELECTRODIALYSIS USING BIPOLAR MEMBRANES

(84) Etats contractants désignés:
**AT BE CH DE DK ES FR GB GR IE IT LI LU NL PT SE**

(30) Priorité: **18.01.1995 FR 9500499**

(43) Date de publication de la demande: **05.11.1997 Bulletin 1997/45**

(73) Titulaire: **ELF ATOCHEM S.A.**
**92800 Puteaux, Hauts-de-Seine (FR)**

(72) Inventeurs:
- **GAVACH, Claude**
  **F-34430 Saint-Jean-de-Vedas (FR)**
- **GANCET, Christian**
  **F-64140 Lons (FR)**
- **MIRASSOU, Alfred**
  **F-64230 Poey (FR)**
- **PERIE, Frédéric**
  **F-64140 Billere (FR)**

(74) Mandataire: **Michardière, Bernard**
**7ter Boulevard Henri Ruel**
**94120 Fontenay-sous-Bois (FR)**

(56) Documents cités:
**EP-A- 0 439 636**
**EP-A- 0 492 209**
**WO-A-88/07975**
**WO-A-90/06167**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 804 277 B1

Printed by Xerox (UK) Business Services
2.16.6/3.4

## Description

La présente invention concerne le domaine de l'électrodialyse et a plus particulièrement pour objet la régénération d'acides à partir de leurs sels par électrodialyse avec membranes bipolaires.

Les acides et les bases sont des intermédiaires importants pour la fabrication d'un grand nombre de produits chimiques. Après leur utilisation, ces acides et ces bases se retrouvent généralement sous forme de solutions aqueuses salines dont il faut se débarrasser. Pour des raisons d'environnement et d'économie, il est souhaitable de régénérer directement les acides et bases de départ à partir des sels contenus dans ces effluents industriels.

L'électrodialyse avec membranes bipolaires permet d'opérer une telle régénération. Cette méthode connue utilise l'énergie électrique pour dissocier l'eau de la solution saline et reconstituer séparément l'acide et la base selon la réaction :

$$\underset{\text{sel}}{MX} + H_2O \rightarrow \underset{\text{acide}}{HX} + \underset{\text{base}}{MOH}$$

Afin de réaliser cette réaction et de maintenir les espèces séparées, on utilise des membranes échangeuses d'ions et, plus particulièrement, des membranes bipolaires constituées de deux faces sélectives respectivement aux anions et aux cations. Sous l'influence d'un champ électrique, ces membranes permettent la réaction suivante :

$$H_2O \rightarrow H^+ + OH^-$$

Les ions $H^+$ et $OH^-$ sont alors réassociés respectivement aux anions $X^-$ et cations $M^+$ provenant du sel, et les espèces obtenues sont maintenues séparées par des membranes échangeuses d'ions classiques (monopolaires) dans une cellule à trois compartiments.

Ce procède de régénération de sels en acides et en bases a déjà été appliqué à de nombreux cas, par exemple :

- acide sulfurique à partir de $Na_2SO_4$ JP-A--4-132605 et l'article de S.SRIDHAR "Elektrodialyse mit bipolaren Membranen" dans Chem. Ing. Tech. 61 (1989) N°5, pp. 428-429) ;
- acide chlorhydrique à partir de NaCl [résumés Chemical Abstracts 117(16) : 153850m, 109(2) : 11524u, 92(6) : 43961f, et 92(4) : 25083s] ;
- acide butyrique à partir de son sel de sodium [resumé CA 116(18) : 182124n] ;
- acide maléïque à partir de son sel d'ammonium (article de S.SRIDHAR précité) ;
- acide borique à partir de borates [résumés CA 107(4) : 29755p et CA 114(4) : 30673m] ;
- acide tartrique à partir de son sel de potassium (demande de brevet FR-A-2 646 421) ;
- acides sulfoniques organiques [résumé CA 71(12) : 56451f et brevet US-A-5 221 443].

Les membranes bipolaires disponibles actuellement sur le marché présentent des performances variables selon la technologie utilisée pour les fabriquer et selon le fournisseur. De par leur nature, les membranes bipolaires sont en principe non perméables aux cations et aux anions qui sont respectivement arrêtés par les couches échangeuse d'anions et échangeuse de cations de la membrane bipolaire.

Les inventeurs ont constaté que, lors de la régénération d'acides organiques forts à partir de leurs sels, par électrodialyse avec membranes bipolaires, il se produisait une contamination de l'acide obtenu par le cation du sel et, en particulier, par le sodium. Cette contamination, variable suivant le niveau de concentration de la base choisi pour travailler, est très gênante lorsque l'on souhaite obtenir un acide de pureté élevée. Ainsi, pour la régénération d'acide méthanesulfonique (AMS) à partir de solutions aqueuses de mésylates, cette contamination constitue un obstacle très important lorsque l'AMS doit ensuite être concentré à un niveau élevé ; le sel présent dans l'acide se concentre également et, pour une certaine valeur de concentration, il y a précipitation et prise en masse d'un mélange ternaire AMS-sel d'AMS-eau.

Les inventeurs ont déterminé que la contamination de l'acide en cation alcalin provient du compartiment base, séparé du compartiment acide par la membrane bipolaire, et non du compartiment sel séparé du compartiment acide par une membrane échangeuse d'anions monopolaire.

Les inventeurs ont ainsi trouvé, qu'en réalité, les membranes bipolaires utilisées jusqu'à maintenant présentent une fuite en cations, en particulier en sodium, provoquant la contamination de l'acide lors de l'opération de régénération.

Les inventeurs ont ensuite trouvé que la perméabilité des membranes bipolaires aux cations, en particulier au

sodium, peut être considérablement diminuée, sans contre-effet sensible sur le procédé d'électrodialyse, en ajoutant une membrane échangeuse d'anions sur la face échangeuse d'anions des membranes bipolaires. Les membranes échangeuses d'anions présentent un nombre de transport très faible pour les cations et constituent de ce fait des barrières très efficaces pour limiter leur diffusion. L'assemblage d'une membrane échangeuse d'anions A,10 et d'une membrane bipolaire BP, tel que schématisé à la Figure 1 fonctionne comme une cellule d'électrodialyse à membranes bipolaires.

L'invention a donc pour objet un procédé de régénération d'acides, en particulier d'acides organiques forts, à partir de leurs sels, par électrodialyse avec membrane bipolaire, caractérisé en ce que l'on applique au moins une membrane échangeuse d'anions supplémentaire contre la face échangeuse d'anions, limitant un compartiment base, de la membrane bipolaire, de manière à réduire la contamination du compartiment acide, situé de l'autre côté de la membrane bipolaire, par des cations.

On réalise une étanchéité suivant tout le pourtour entre la membrane échangeuse d'anions et la membrane bipolaire.

Comme membrane échangeuse d'anions, on peut utiliser n'importe quelle membrane échangeuse d'anions du commerce, par exemple celles commercialisées par la Société Asahi Glass sous la dénomination SELEMION®, par la Société Tokuyama Soda sous la dénomination NEOSEPTA® ou par la Société Solvay . Ces membranes commerciales ont en général une épaisseur comprise entre 0,1 et 1 mm et un diamètre de pore compris entre 1 et 30 $\mu$m. Les membranes échangeuses d'anions sont habituellement composées d'une matrice polymérique, telle que polystyrène-divinylbenzène, contenant des groupements cationiques chimiquement liés (par exemple ammonium ou ammonium substitué), alors que les membranes échangeuses de cations portent des groupements carboxylate ou sulfonate.

Bien que le procédé selon l'invention vise plus particulièrement la régénération de l'acide méthanesulfonique à partir de ses sels alcalins, en particulier du sel de sodium, il peut généralement s'appliquer à la régénération d'acides organiques forts comme les acides sulfoniques et les acides phosphoniques, pourvu que leur masse moléculaire ne soit pas trop importante.

L'électrodialyse proprement dite est effectuée dans les conditions habituelles connues de l'homme du métier.

L'invention est également relative à un dispositif d'électrodialyse avec membrane bipolaire, pour la mise en oeuvre du procédé, constitué d'un empilement de membranes échangeuses de cations, bipolaires, échangeuses d'anions, avec interposition de séparateurs et de joints d'encadrement, entre deux plaques de serrage appliquées contre des porte-électrode et caractérisé par le fait qu'il comporte, au niveau de chaque membrane bipolaire une membrane échangeuse d'anions supplémentaire appliquée contre la face échangeuse d'anions de la membrane bipolaire, cette membrane supplémentaire échangeuse d'anions et la membrane bipolaire étant serrées de manière étanche, suivant tout leur pourtour, entre deux joints d'encadrement de telle sorte qu'aucun espace mesurable ne subsiste entre les faces en contact de la membrane échangeuse d'anions supplémentaire et de la membrane bipolaire.

De préférence, tous les éléments de l'empilement, y compris la membrane bipolaire et la membrane échangeuse d'anions supplémentaire ont mêmes contours extérieurs et comportent, a leur périphérie, des orifices d'écoulement des fluides.

L'invention sera mieux comprise à l'aide d'exemples de réalisation, décrits avec référence aux dessins ci-annexés, mais qui ne sont nullement limitatifs.

La Figure 1, de ces dessins, est un schéma d'assemblage d'une membrane échangeuse d'anions et d'une membrane bipolaire selon l'invention, schéma dans lequel la membrane échangeuse d'anions à été représentée, dans un but explicatif, écartée de la face échangeuse d'anions de la membrane bipolaire, alors qu'en réalité la membrane échangeuse d'anions est plaquée contre cette face anionique.

La Figure 2 est un schéma simplifié de cellules d'électrodialyse d'un type connu.

La Figure 3 est un schéma, semblable à celui de la Figure 2, de cellules d'électrodialyse selon l'invention ;

La Figure 4 est un schéma simplifié, en perspective, du montage d'une membrane bipolaire et d'une membrane échangeuse d'anions, selon l'invention, entre deux joints d'encadrement.

La Figure 5 est une coupe d'un empilement de compartiments formant deux cellules.

La Figure 6 est une vue de face d'un séparateur de compartiment.

La Figure 7, enfin, est une coupe suivant la ligne VII-VII de la Figure 6.

En se reportant aux dessins, on peut voir que, selon l'invention, une membrane échangeuse d'anions A,10 ( ou simplement 10 ) est assemblée a une membrane bipolaire BP en étant étroitement appliquée contre la face échangeuse d'anions de la membrane bipolaire BP. Sur la Figure 1 un écart a été représenté entre la membrane échangeuse d'anions A,10 et la face échangeuse d'anions de la membrane bipolaire BP pour montrer que la membrane échangeuse d'anions A,10 est une membrane supplémentaire rapportée. En réalité, il n'y a aucun espace entre cette membrane échangeuse d'anions rapportée et la membrane bipolaire. La membrane échangeuse d'anions A,10 est appliquée étroitement contre la face échangeuse d'anions de la membrane bipolaire BP et il n'y a aucune circulation de fluide entre ces membranes, parallèlement à leur plan moyen, contrairement à ce que se passe dans les divers compartiments d'une cellule d'électrodialyse.

Avant de décrire plus en détail l'assemblage de la membrane échangeuse d'anions supplémentaire et de la membrane bipolaire, avec référence aux Figures 4 à 7, on va considérer un certain nombre d'exemples.

EXEMPLES

Les exemples suivants que illustrent l'invention sans la limiter, ont été réalisés dans un dispositif de type classique comprenant trois circuits pour les réactifs (sel, acide, base) et un circuit pour l'électrolyte (NaOH 2,5 N).

Le volume du réservoir de chaque circuit était d'environ 8 litres et la structure de l'empilement utilisé, schématisée à la Figure 2 où les signes et lettres ont les significations suivantes :

| | |
|---|---|
| - + | membrane bipolaire |
| - | membrane échangeuse d'anions |
| + | membrane échangeuse de cations |
| N | membrane Nafion (échangeuse de cations) |
| S | compartiment sel |
| A | compartiment acide |
| B | compartiment base |
| ER | rinçage électrodes |

correspondait à 4 cellules pour une surface totale de 0,04 m2.

Les paramètres généraux utilisés étaient les suivants :

- tension maximale (pour cet empilement) : 20 volts
- courant maximal (pour cet empilement) : 10 ampères
- débit de l'électrolyte (par électrode) : 50 l/h
- débit de chaque réactif : 90 l/h
- pression a l'entrée de l'empilement : 0,4-0,5 bar

EXEMPLE 1 (Comparatif)

A titre de comparaison, un premier traitement par électrodialyse d'une solution d'AMS avec membrane bipolaire, a été effectué a l'aide de cellules d'électrodialyse classiques illustrées sur la Figure 2. Le compartiment acide A est délimité, d'un côté, par la face échangeuse de cations d'une membrane bipolaire BP classique et, de l'autre côté, par une membrane monopolaire échangeuse d'anions. De l'autre côté de la membrane bipolaire BP se trouve le compartiment base B que est donc délimité par la face échangeuse d'anions de la membrane bipolaire et par une membrane monopolaire échangeuse de cations. De l'autre côté de la membrane échangeuse d'anions délimitant le compartiment acide A se trouve le compartiment sel S délimité par une membrane échangeuse de cations établissant une séparation entre le compartiment sel S et le compartiment base B. Les écarts entre les membranes suivant une direction perpendiculaire à leur plan moyen sont suffisants pour permettre une circulation de liquide dans les compartiments en question.

Le dispositif selon le schéma de la Figure 2 a été équipé avec 4 membranes bipolaires WSI fournies par WSI Technologies Inc., 5 membranes échangeuses de cations (2 membranes Nafion de DuPont de Nemours et 3 membranes CMV d'Asahi Glass) et a membranes échangeuses d'anions AAV d'Asahi Glass.

Le compartiment sel a été chargé avec 4 litres d'une solution de mésylate de sodium à régénérer (210 g/l et pH 3,0) et les compartiments acide et base ont été chargés respectivement avec 3 litres d'une solution 0,56N d'AMS contenant 150 ppm de $Na^+$ et 3 litres d'une solution

0,53N d'hydroxyde de sodium. Les débits de circulation étaient fixés à 90l/h, l'intensité a 10A et la tension ajustée entre 16 et 20V pour obtenir cette intensité.

Apres 5 heures de fonctionnement, la concentration de a solution d'AMS est montée à 1,62N pour un volume final de 3,5 litres et celle de la solution. de NaOH a 2,16N. Pendant cette durée de fonctionnement, la teneur en sodium dans la solution d'AMS est passée de 150 à 1000 ppm, ce qui correspond à la diffusion de 3025 mg de sodium pendant la durée de l'essai.

EXPERIENCE MONTRANT L'ORIGINE DU SODIUM PRESENT DANS L'AMS.

Après avoir constaté la contamination de l'acide par le sodium, selon cet exemple 1, les inventeurs se sont efforcés d'en déterminer l'origine.

L'expérience suivante a été effectuée.

En reprenant les conditions de l'exemple 1, on a fait circuler dans le compartiment sel S une solution de NaMS

(mésylate de sodium) tandis que dans le compartiment base B on a fait circuler une solution d'hydroxyde de potassium KOH, à la place de l'hydroxyde de sodium.

On a suivi, en l'absence de courant électrique, la diffusion des cations dans le compartiment acide A. A l'instant t = 0, les concentrations respectives dans l'AMS du compartiment acide A était de 106 ppm pour $Na^+$, et de 1 ppm pour $K^+$.

Après cinq heures de circulation des solutions dans les compartiments, les concentrations sont devenues respectivement de 107 ppm pour $Na^+$ et de 129 ppm pour $K^+$ dans le compartiment acide A.

Autrement dit, la concentration dans le compartiment acide A en cations $Na^+$ n'a pratiquement pas varié, et ces cations que ne peuvent provenir que du compartiment sel S n'ont donc pas traversé la membrane monopolaire échangeuse d'anions séparant le compartiment acide du compartiment sel.

Par contre, la concentration en cations $K^+$ dans le compartiment acide A a considérablement augmenté. Ces cations $K^+$ ne peuvent provenir que du compartiment base B séparé du compartiment acide par la membrane bipolaire BP.

Cette expérience montre donc que la contamination du compartiment acide A en cations alcalins est due à une certaine perméabilité de la membrane bipolaire BP à l'égard de ce cation alcalin.

Ayant ainsi trouvé la source de la contamination, les inventeurs ont imaginé de renforcer l'imperméabilité aux cations de la membrane bipolaire BP en appliquant contre la face échangeuse d'anions de la membrane bipolaire BP une membrane échangeuse d'anions supplémentaire comme illustrée sur la Figure 3.

Le schéma de la Figure 3, que est celui de cellules d'électrodialyse selon l'invention, correspond dans son ensemble au schéma classique de la Figure 2 avec pour différence essentielle la mise en place contre la face échangeuse d'anions de chaque membrane bipolaire BP d'une membrane échangeuse d'anions supplémentaire 10.

Une étanchéité est réalisée suivant tout le contour des membranes BP et 10 appliquées l'une contre l'autre ; il n'y a pas de circulation de liquide entre ces membranes.

Les expériences décrites dans les exemples 2 et 3 que suivent ont alors été effectuées dans les conditions précisées ci-après.

EXEMPLE 2

On a utilisé le même dispositif qu'à l'exemple 1, mais en appliquant une membrane échangeuse d'anions 10 (Figure 3) AMV d'Asahi Glass sur la face échangeuse d'anions de chacune des 4 membranes bipolaires.

Le compartiment sel a été chargé avec 4 litres d'une solution de mésylate de sodium (210 g/l et pH 2,9) et les compartiments acide et base ont été chargés respectivement avec 3 litres d'une solution 0,7N d'AMS contenant 73 ppm de $Na^+$ et 3 litres d'une solution 0,5N de NaOH.

Pour une tension de 20V appliquée, l'intensité observée s'est située entre 9 et 1OA. Après 5 heures de fonctionnement, la concentration de la solution d'AMS est montée a 1,75N pour un volume final de 3,4 litres et celle de la solution de NaOH à 1,73N. Dans le même temps, la teneur en sodium dans l'AMS est passée de 73 à 316 ppm, ce que correspond à a diffusion de 852 mg de sodium.

Par rapport a l'exemple 1, on note une diffusion des cations $Na^+$ beaucoup plus faible (division par un facteur supérieur à 3).

EXEMPLE 3

On a opéré comme a l'exemple 2, mais les membranes AMV utilisées pour renforcer la couche échangeuse d'anions des membranes bipolaires ont été remplacées par des membranes échangeuses d'anions ADP de Solvay.

Les concentrations d'AMS et de NaOH étaient respectivement de 0,58N et 0,57N au départ, et de 1,46N et 1,62N après 5 heures de fonctionnement sous une tension de 20V, l'intensité moyenne observée étant de 6-8A. Le volume final d'AMS était de 3,3 litres.

Pendant l'essai, la teneur en sodium de l'AMS est passée de 56 à 123 ppm, ce que correspond à la diffusion de 218 mg de sodium en 5 heures. Ceci est environ 4 fois plus faible qu'à l'exemple 2 et près de 14 fois plus faible qu'à l'exemple 1.

Le tableau suivant récapitule l'ensemble des résultats des exemples 1 à 3.

| Exemple | Dispositif | Fuite en $Na^+$ en 5h (mg) | AMS production en 5h (moles) | Na/AMS (mg/mole) |
|---|---|---|---|---|
| 1 | Membranes bipolaires WSI seules | 3025 | 4,0 | 756 |
| 2 | Membranes WSI + Membranes échangeuses d'anions AMV plaquées sur la face échangeuse d'anions des bipolaires | 852 | 3,8 | 224 |
| 3 | Membranes WSI + Membranes échangeuses d'anions ADP plaquées sur la face échangeuse d'anions des bipolaires | 218 | 3,1 | 70 |

Pour une description plus précise des membranes utilisées dans les exemples précédents, on pourra se reporter au Tome 1 "Membranes d'électrodialyse" de l'ouvrage intitulé "MEMBRANES SEMI-PERMEABLES CHARGEES ET SEPARATEURS ELECTROCHIMIQUES" édité en 1991 par EDF (Les Renardières, BP 1, 77250 MORET-SUR-LOING). La référence attribuée par cet ouvrage aux différentes membranes est indiquée dans le tableau suivant :

| Membranes | Références |
|---|---|
| AAV | ED A-07 |
| AMV | ED A-15 |
| ADP | ED M-11 |
| CMV | ED A-27 |
| Nafion | SD D-33 |
| WSI | ED W-01 |

En se reportant aux Figures 4 à 7 on peut voir un dispositif pour la mise en oeuvre du procédé de l'invention.

La Figure 4 montre schématiquement la préparation d'une membrane bipolaire BP dont la face échangeuse d'anions 11 est renforcée par une membrane échangeuse d'anions 10 selon l'invention. Comme il résulte de la Figure 4, la membrane échangeuse d'anions supplémentaire 10 est appliquée contre la face échangeuse d'anions 11, sans laisser d'espace mesurable entre les deux faces en contact. La membrane bipolaire BP et la membrane échangeuse d'anions supplémentaire associée 10, de forme rectangulaire dans l'exemple représenté, sont serrées, suivant tout leur pourtour, entre deux joints d'encadrement 12 de forme semblable à celle des membranes, comportant une ouverture centrale 13, également rectangulaire. Cette ouverture 13 découvre la majeure partie de la face de la membrane 10 située du côté opposé a la membrane bipolaire BP, et de la face échangeuse de cations de cette membrane BP. Le contour fermé des joints d'encadrement 12 comporte plusieurs orifices 14 de circulation de fluide, séparés les uns des autres. L'empilement d'orifices de plusieurs éléments permet de créer des canaux de circulation de fluide isolés les uns des autres.

Dans l'exemple simplifié de la Figure 4, la membrane bipolaire BP et la membrane monopolaire échangeuse d'anions 10 ont été représentées avec un contour de dimensions inférieures a celui des joints d'encadrement 12, de sorte que ces membranes ne comportent pas d'orifices de circulation de fluide semblables aux orifices 14.

Toutefois, comme montré dans la réalisation de la Figure 5, les membranes BP et 10 peuvent avoir des dimensions extérieures identiques à celles des joints d'encadrement 12 et comporter des orifices semblables aux orifices 14 que viennent s'aligner avec ceux des joints d'encadrement 12 et ceux des autres éléments de l'empilement.

Cet empilement de la figure 5 comprend, de la droite vers la gauche : un flasque de serrage 15 ; un porte-électrode 16 ; une électrode à savoir une anode 17 logée dans le porte-électrode 16 ; un premier joint d'encadrement 12 appliqué contre le porte-électrode 16 ; un séparateur d'électrode 18 ; à nouveau un joint d'encadrement 12 ; une membrane échangeuse de cations 19 ; un joint d'encadrement 12 ; un séparateur de compartiment 20B, correspondant au compartiment base ; un joint d'encadrement 12 ; la membrane échangeuse d'anions supplémentaire 10 appliquée contre la face échangeuse d'anions de la membrane bipolaire BP ; un joint d'encadrement 12 ; un séparateur de compartiment 20A correspondant au compartiment acide ; un joint d'encadrement 12 ; une membrane échangeuse d'anions monopolaire 21 ; un joint d'encadrement 12 ; un séparateur de compartiment 20S correspondant au compartiment sel ; un joint d'encadrement 12 ; à nouveau une membrane échangeuse de cations 19. On retrouve ensuite la succession d'éléments indiqués ci-dessus permettant de constituer un compartiment base suivi d'un compartiment acide et d'un compartiment sel.

Une cellule d'électrodialyse correspond à l'ensemble de trois compartiments sel, base et acide.

A l'extrémité gauche de l'empilement on trouve un porte-électrode 22 dans lequel est logée l'autre électrode, à savoir la cathode 23. Une autre plaque de serrage 15 est appliquée contre le porte-électrode 22, du côté opposé à l'empilement.

L'ensemble des éléments en forme de plaques de l'empilement ont tous un contour rectangulaire extérieur identique et comportent des orifices 14 répartis sur leur périphérie, aux mêmes endroits, pour constituer des canaux de circulation.

Le porte-électrode 16 et le porte-électrode 22 sont munis de conduits tels que 24, 25 propres à communiquer avec un canal formé par un empilement d'orifices 14. Le conduit 24 constitue l'arrivée d'acide a régénérer dans l'exemple considéré, tandis que le conduit 25 permet la sortie de l'acide régénéré.

Comme visible sur la Figure 6, chaque élément séparateur de compartiment, par exemple l'élément 20A pour le compartiment acide, est formé par une plaque d'une épaisseur déterminée comportant au centre une ouverture rectangulaire 26 munie d'une grille 27 assurant la diffusion du liquide sans en empêcher la circulation. L'ouverture 26 est reliée, par exemple suivant un segment de sa tranche, à un orifice 14 par une partie évidée 28, à contour trapézoïdal, réalisée dans l'épaisseur de la plaque formant l'élément 20A. Cette partie évidée 28 comporte une membrane 29 à canaux intérieurs pour la circulation du fluide.

Une autre zone du contour de l'ouverture 26, par exemple son angle supérieur droit sur la Figure 6, est reliée, par une autre partie évidée 30 de l'élément séparateur 20A, à un orifice 14 servant a l'évacuation du liquide. La partie évidée 30 comporte également une membrane 29 à canaux intérieurs pour la circulation du liquide.

On voit ainsi qu'en choisissant la position des parties évidées 28, 30 on peut mettre en communication la chambre correspondant à l'ouverture 27 avec des orifices 14 différents et donc avec des canaux différents. Ainsi, on peut réaliser un circuit pour l'acide avec des éléments tels que 20A, tandis que pour le sel les éléments 20S comportent des parties évidées placées différemment, de même pour les éléments 20B relatifs au compartiment base.

Comme visible sur la Figure 5, dans un compartiment acide, compris entre la face échangeuse de cations de la membrane bipolaire BP et la membrane monopolaire échangeuse d'anions 21, la dimension de la chambre, suivant la direction de l'axe de l'empilement, est égale a la somme de deux épaisseurs de joint d'encadrement 12 et de l'épaisseur de l'élément séparateur 20A.

L'entrée de l'acide a régénérer et sa sortie ne peuvent s'effectuer que par les membranes à canaux 29 situées dans les évidements 28 et 30 de l'élément séparateur 20A, entre les joints d'encadrement 12. Les flèches tracées sur la Figure 5 permettent de suivre la circulation de l'acide.

Une circulation semblable a lieu pour le sel et la base dans des canaux différents.

On voit que contrairement au cas des compartiments acide, base et sel dans lesquels une distance non négligeable existe entre les faces des membranes en regard limitant ces compartiments, il n'y a aucun espace entre la face échangeuse d'anions de la membrane bipolaire BP et la membrane échangeuse d'anions supplémentaire 10. Ces deux membranes sont appliquées de manière étanche suivant leur pourtour selon la pression appliqueé sur l'ensemble de l'empilement par le serrage des porte-électrode 15.

Le montage de la membrane échangeuse d'anions supplémentaire 10, effectué par plaquage, constitue une solution simple et économique pour renforcer l'effet barrière pour les cations au niveau de la membrane bipolaire et réduire considérablement la contamination par les cations de l'acide régénéré.

Comme déjà indiqué toute membrane échangeuse d'anions peut convenir pour ce renforcement de l'effet barrière. Toutefois une membrane à taux de réticulation plus élevé, comme l'ADP, donne de meilleurs résultats qu'une membrane échangeuse d'anions standard comme l'AMV.

## Revendications

1. Procédé d'électrodialyse avec membrane bipolaire pour la régénération d'acides à partir de leurs sels, procédé selon lequel on réalise entre une électrode positive (anode) et une électrode négative (cathode) une succession de

compartiments base, acide, sel, déterminés par une succession de membranes échangeuse de cations, bipolaire, échangeuse d'anions, échangeuse de cations, bipolaire, etc ..., une circulation de solution ayant lieu dans chaque compartiment, caractérisé par le fait qu'on applique contre la face échangeuse d'anions (11), limitant un compartiment base, de la membrane bipolaire (BP) au moins une membrane échangeuse d'anions supplémentaire (10), sans laisser d'écart mesurable entre les deux membranes, de manière à réduire la contamination, par des cations, du compartiment acide situé de l'autre côté de la membrane bipolaire (BP).

**2.** Procédé selon la revendication 1, caractérisé par le fait qu'on réalise une étanchéité entre la membrane échangeuse d'anions supplémentaire (10) et la membrane bipolaire (BP) suivant tout leur pourtour.

**3.** Procédé selon la revendication 1 ou 2, caractérisé par le fait que la membrane échangeuse d'anions supplémentaire (10) est une membrane à taux de réticulation élevé.

**4.** Procédé selon l'une des revendications 1 à 3 caractérisé en ce que l'on régénère des acides organiques forts.

**5.** Procédé selon l'une des revendications 1 à 4, caracterisé en ce que l'on régénère de l'acide méthanesulfonique.

**6.** Dispositif d'électrodialyse avec membrane bipolaire pour la mise en oeuvre du procédé selon l'une des revendications 1 à 3, constitué d'un empilement de membranes échangeuses de cations (19), bipolaires (BP), échangeuses d'anions (21), avec interposition de séparateurs et de joints d'encadrement (12,18,20A,20B,20S), entre deux plaques de serrage (15) appliquées contre des porte-électrode (16,22), caractérisé par le fait qu'il comporte, au niveau de chaque membrane bipolaire (BP) une membrane échangeuse d'anions supplémentaire (10) appliquée contre la face échangeuse d'anions (11) de la membrane bipolaire, cette membrane supplémentaire échangeuse d'anions (10) et la membrane bipolaire (BP) étant serrées de manière étanche, suivant tout leur pourtour, entre deux joints d'encadrement (12) de telle sorte qu'aucun espace mesurable ne subsiste entre les faces en contact de la membrane échangeuse d'anions supplémentaire (10) et de la membrane bipolaire (BP).

**7.** Dispositif selon la revendication 6, caractérisé par le fait que tous les éléments de l'empilement, y compris la membrane bipolaire (BP) et la membrane échangeuse d'anions supplémentaire (10) ont mêmes contours extérieurs et comportent, à leur périphérie, des orifices (14) d'écoulement des fluides.

**8.** Dispositif selon la revendication 6 ou 7, caractérisé par le fait que chaque compartiment acide, base ou sel comporte un séparateur de compartiment (20A, 20B, 20S) propre à relier, entre deux joints d'encadrement (12), le compartiment à un canal d'arrivée et à un canal de sortie.

**9.** Dispositif selon l'une des revendications 6 à 8, caractérisé par le fait que chaque joint d'encadrement (12) comporte une ouverture centrale (13) dégageant les faces de la ou des membranes serrées entre deux joints (12).

## Claims

**1.** Electrodialysis process, using a bipolar membrane, for the regeneration of acids from their salts, according to which process a succession of base, acid and salt compartments is produced between a positive electrode (anode) and a negative electrode (cathode), these Compartments being defined by a succession of membranes, namely a cation-exchange membrane, a bipolar membrane, an anion-exchange membrane, a cation-exchange membrane, a bipolar membrane, etc., solution flowing through each compartment, characterized in that at least one additional anion-exchange membrane (10) is applied against the anion-exchange face (11), delimiting a base compartment, of the bipolar membrane (BP) without leaving a measurable gap between the two membranes, so as to reduce the contamination, by cations, of the acid compartment lying on the other side of the bipolar membrane (BP).

**2.** Process according to Claim 1, characterized in that sealing is provided between the additional anion-exchange membrane (10) and the bipolar membrane (BP) around their entire perimeter.

**3.** Process according to Claim 1 or 2, characterized in that the additional anion-exchange membrane (10) is a membrane having a high degree of crosslinking.

**4.** Process according to one of Claims 1 to 3, characterized in that strong organic acids are regenerated.

**5.** Process according to one of Claims 1 to 4, characterized in that methanesulfonic acid is regenerated.

6. Electrodialysis device, using a bipolar membrane for implementation of the process according to one of Claims 1 to 3, consisting of a stack of cation-exchange (19), bipolar (BP) and anion-exchange (21) membranes, with interposition of separators and frame gaskets (12, 18, 20A, 20B, 20S) between two clamping plates (15) applied against electrode supports (16, 22), characterized in that it includes, at each bipolar membrane (BP), an additional anion-exchange membrane (10) applied against the anion-exchange face (11) of the bipolar membrane, this additional anion-exchange membrane (10) and the bipolar membrane (BP) being clamped in a sealed manner, around their entire perimeter, between two frame gaskets (12) in such a way that no measurable space remains between the contacting faces of the additional anion-exchange membrane (10) and of the bipolar membrane (BP).

7. Device according to Claim 6, characterized in that all the elements in the stack, including the bipolar membrane (BP) and the additional anion-exchange membrane (10), have the same outer profiles and have, on their periphery, holes (14) for fluid flow.

8. Device according to Claim 6 or 7, characterized in that each acid, base or salt compartment includes a compartment separator (20A, 20B, 20S) suitable for connecting, between two frame gaskets (12), the compartment to an inlet channel and to an outlet channel.

9. Device according to one of Claims 6 to 8, characterized in that each frame gasket (12) has a central opening (13) which exposes the faces of the membrane or membranes clamped between two gaskets (12).

**Patentansprüche**

1. Elektrodialyseverfahren mit bipolarer Membran zur Regenerierung von Säuren ausgehend von ihren Salzen, bei dem man zwischen einer positiven Elektrode (Anode) und einer negativen Elektrode (Kathode) eine Abfolge von Zellen (Kompartimenten) mit Base, Säure oder Salz vorsieht, bestimmt durch ein Aufeinanderfolgen von Kationenaustauschmembran, bipolarer Membran, Anionenaustauschmembran, Kationenaustauschmembran, bipolarer Membran etc. ..., wobei eine Zirkulation der Lösung in jeder der Zellen stattfindet, dadurch gekennzeichnet, daß man gegen die Anionen-austauschfläche (11) der bipolaren Membran (BP), die die Basenzelle begrenzt, mindestens eine zusätzliche Anionenaustauschmembran (10) vorsieht, ohne einen meßbaren Abstand zwischen den zwei Membranen zu lassen, so daß eine durch Kationen verursachte Verunreinigung der auf der anderen Seite der bipolaren Membran (BP) befindlichen Säurezelle verringert wird.

2. Verfahren nach Anspruch 1, dadurch gekennzeichnet, daß man die Dichtigkeit zwischen der zusätzlichen Anionenaustauschmembran (10) und der bipolaren Membran (BP) über ihren gesamten äußeren Umfang vorsieht.

3. Verfahren nach Anspruch 1 oder 2, dadurch gekennzeichnet, daß die zusätzliche Anionenaustauschmembran (10) eine Membran mit hohem Vernetzungsgrad ist.

4. Verfahren nach einem der Ansprüche 1 bis 3, dadurch gekennzeichnet, daß man starke organische Säuren regeneriert.

5. Verfahren nach einem der Ansprüche 1 bis 4, dadurch gekennzeichnet, daß man Methansulfonsäure regeneriert.

6. Vorrichtung zur Elektrodialyse mit bipolarer Membran zur Durchführung des Verfahrens nach einem der Ansprüche 1 bis 3, bestehend aus einer Abfolge von Kationenaustauschmembranen (19), bipolaren Membranen (BP) und Anionenaustauschmembranen (21) zwischen zwei gegen die Elektrodenträger (16, 22) angeordneten Spannplatten unter Zwischeneinfügung von Trennschichten und Verbindungs- oder Dichtungselementen zur Einfassung (12, 18, 20A, 20B, 20S), dadurch gekennzeichnet, daß die Vorrichtung auf Höhe jeder bipolaren Membran (BP) eine zusätzliche Anionenaustauschmembran (10) umfaßt, die gegen die Anionenaustauschfläche (11) der bipolaren Membran angeordnet ist, wobei die zusätzliche Anionenaustauschmembran (10) und die bipolare Membran (BP) über ihren gesamten äußeren Umfang zwischen zwei Dichtelementen zur Einfassung (12) dicht eingefaßt sind, so daß kein meßbarer Abstand zwischen den Kontaktflächen der zusätzlichen Anionenaustauschmembran (10) und der bipolaren Membran (BP) verbleibt.

7. Vorrichtung nach Anspruch 6, dadurch gekennzeichnet, daß alle Elemente der Abfolge einschließlich der bipolaren Membran (BP) und der zusätzlichen Anionenaustauschmembran (10) selbst äußere Begrenzungsumrisse aufweisen und an ihrem äußeren Rand jeweils Öffnungen (14) für den Zu- und Abfluß von Flüssigkeiten umfassen.

8. Vorrichtung nach Anspruch 6 oder 7, dadurch gekennzeichnet, daß jede Zelle, entweder mit der Säure, der Base oder dem Salz, eine geeignete Zelltrennschicht oder -wand (20A, 20B, 20S) umfaßt, die zwischen zwei Einfassungsdichtungen (12) die Zelle mit einem Zufuhr- und einem Abflußkanal verbindet.

9. Vorrichtung nach einem der Ansprüche 6 bis 8, dadurch gekennzeichnet, daß jede Dichtung zur Einfassung (12) eine mittlere Öffnung (13) umfaßt, die die Flächen der zwischen zwei Dichtungselementen (12) eingefaßten Membran oder Membranen frei läßt.

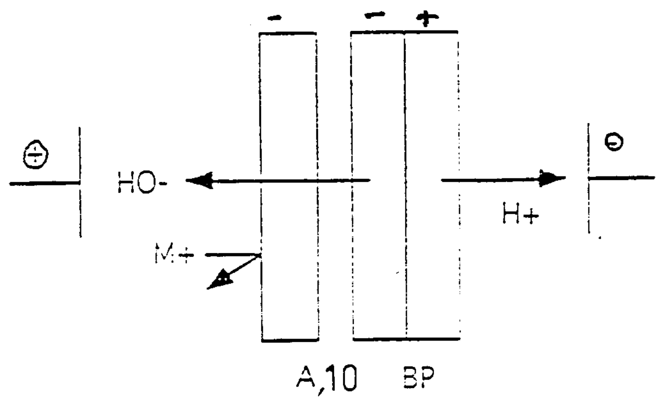


Fig. 1

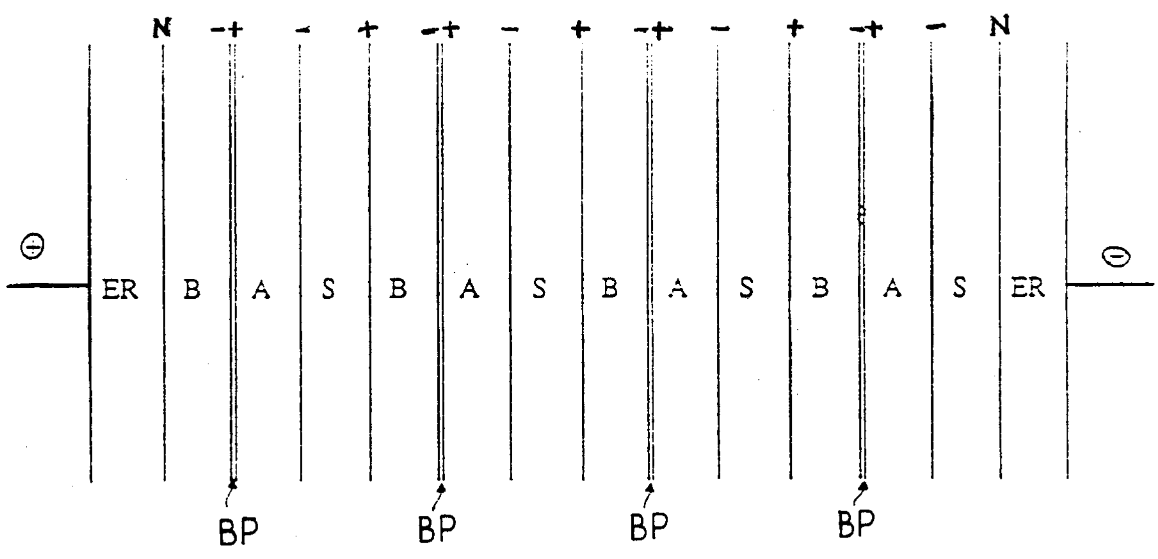


Fig. 2

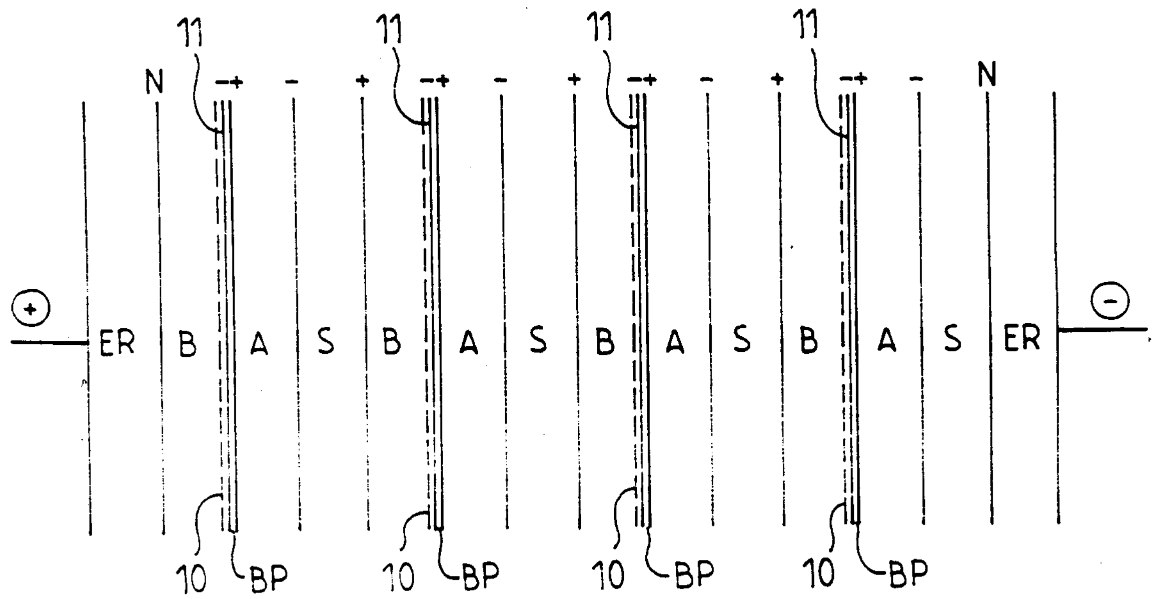
11
11
11
11
N
N
ER
B
A
S
B
A
S
B
A
S
B
A
S
ER
10
BP
10
BP
10
BP
10
BP

FIG.3

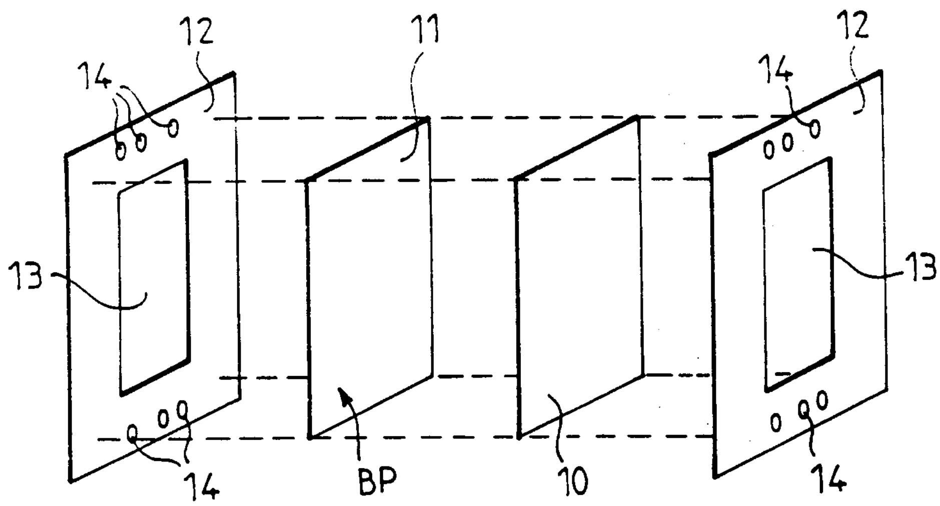
12
11
12
14
14
13
13
14
BP
10
14

FIG.4

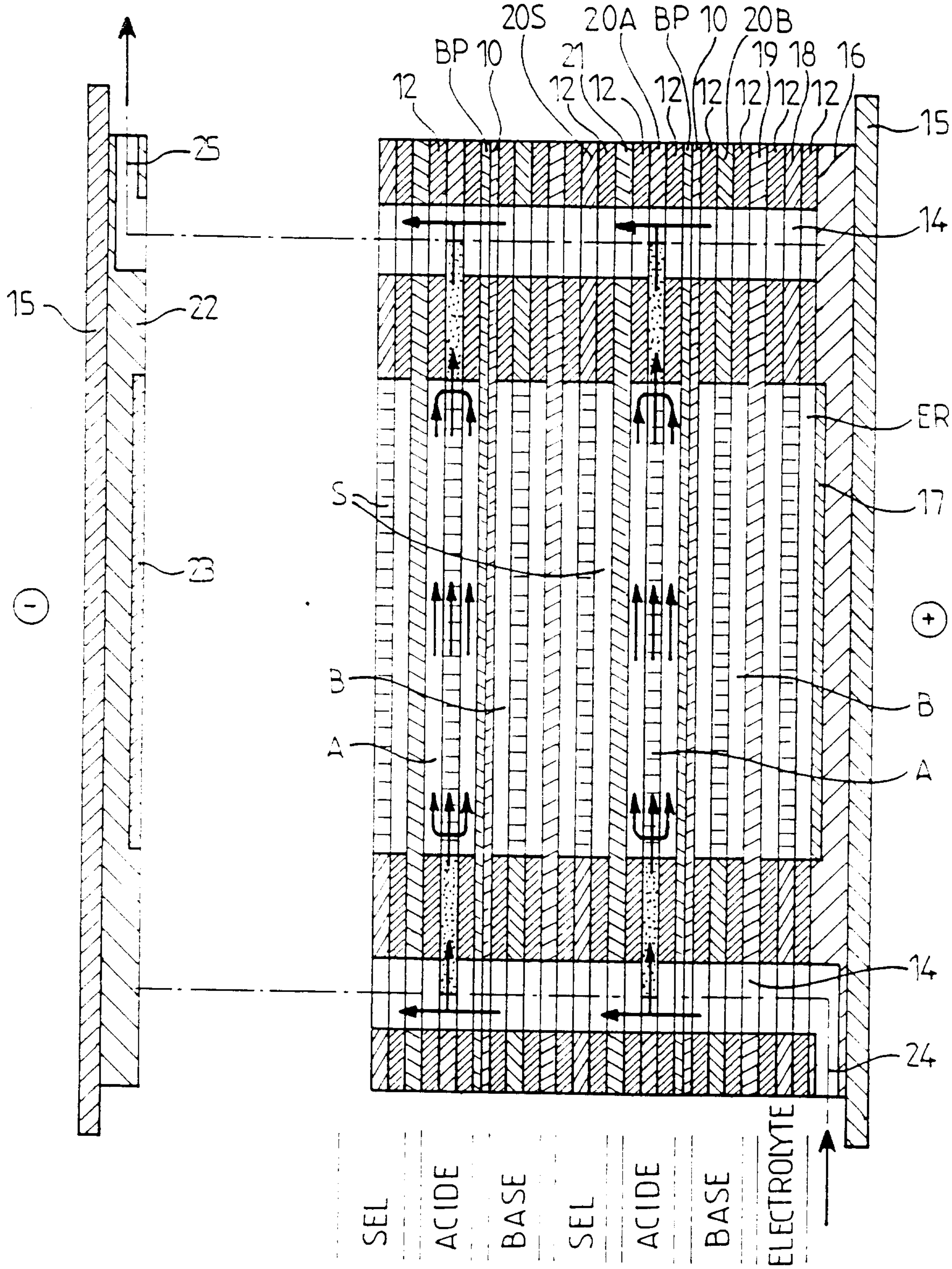
20S
20A
BP 10
20B
BP 10
21
19
18
16
12
25
15
14
22
ER
17
S
23
B
A
24
SEL
ACIDE
BASE
ELECTROLYTE

FIG. 5

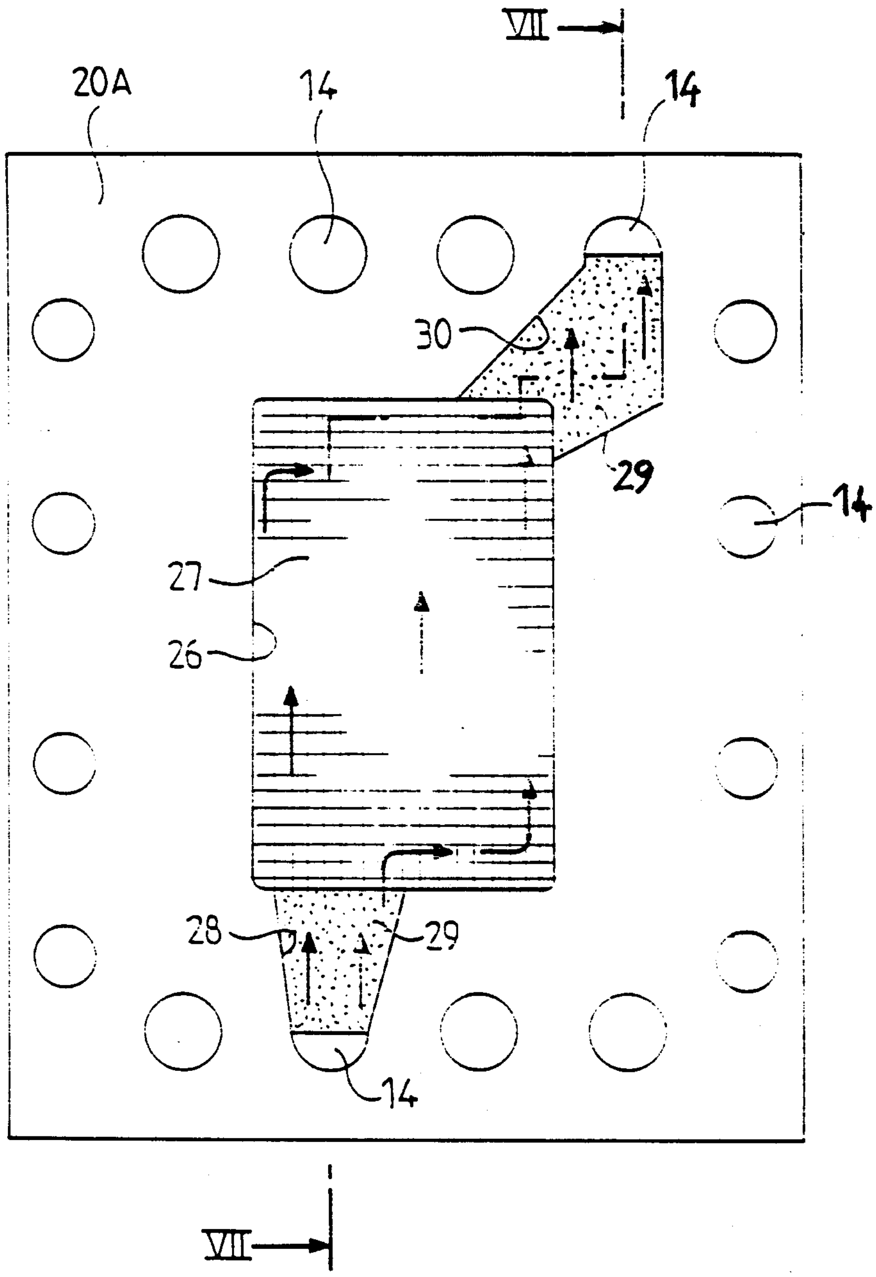


FIG.6

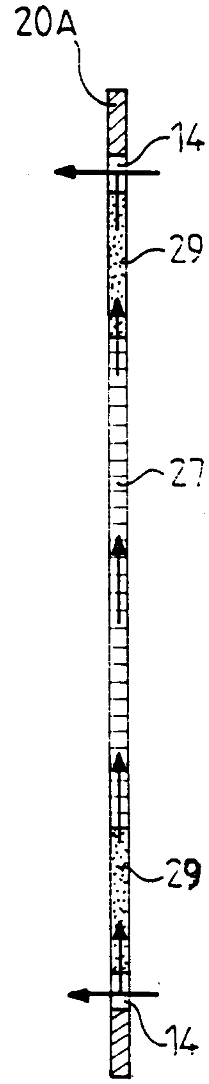


FIG.7